# EUROPEAN PATENT APPLICATION

(11) **EP 1 260 236 A1**
(43) Date of publication of application: **27.11.2002**
(21) Application number: 02253345.9
(22) Date of filing: 14.05.2002
(51) Int. Cl.: A61L 9/03

(54) **Fragrance emitting device**

(30) Priority: 16.05.2001 GB 0111984
(71) Applicant: Pankhurst Design & Developments Limited, London W6 7HJ (GB)
(72) Inventor: Knight, William, London W14 0AL (GB); Groves, Kursty, London W6 7HJ (GB); Hayes-Pankhurst, Richard Paul, London SW6 6LP (GB)
(74) Representative: Knott, Stephen Gilbert

(57) **Abstract**

A fragrance emitting device (10) comprising a container (14) containing two differently coloured, differently fragranced immiscible liquids (46,48), and a light source (28). The light source (28) is located in the base (12) of the device and shines through the container (14) heating a first of said two immiscible liquids (46). Portions (50) of said first immiscible liquid (46) rise from a layer formed by said first immiscible liquid (46) at the bottom of said container (14) into a layer formed immediately above said first layer by said second immiscible liquid (48) thereby heating said second immiscible liquid (48). Vapours (46',48') are released from the device (10) when portions (50) of the first immiscible liquid (46) reach the uppermost surface (52) of the second immiscible liquid (48) and also when the second immiscible liquid (48) reaches a specified temperature. Preferably a replacement container (14) and a cap (16) are removable from the base (12) for easy, inexpensive replacement of said immiscible liquids (46,48).

## Description

The invention relates to a fragrance emitting device.

In known fragrance emitting devices, a fragranced liquid held in a wick or pad is heated to cause partial evaporation of the liquid thereby causing a fragrance to be emitted from the device.

According to a first aspect of the invention, there is provided a fragrance emitting device comprising a heat source and a container containing first and second immiscible liquids, the heat source heating the first immiscible liquid to cause the first liquid to rise from a position at the lower end of the container and pass through the second liquid towards a surface of the second liquid, one of said liquids including a fragrance released from said surface by said heated first liquid.

Preferably, the first liquid contains a fragrance, the first liquid rising to said surface to release the fragrance.

According to a preferred embodiment of the invention, the fragrance emitting device may further comprise a light source with the container comprising at least partially transparent walls so that light from the source passes through the walls.

The following is a more detailed description of a preferred embodiment of the invention, by way of example, reference being made to the accompanying drawings, in which:

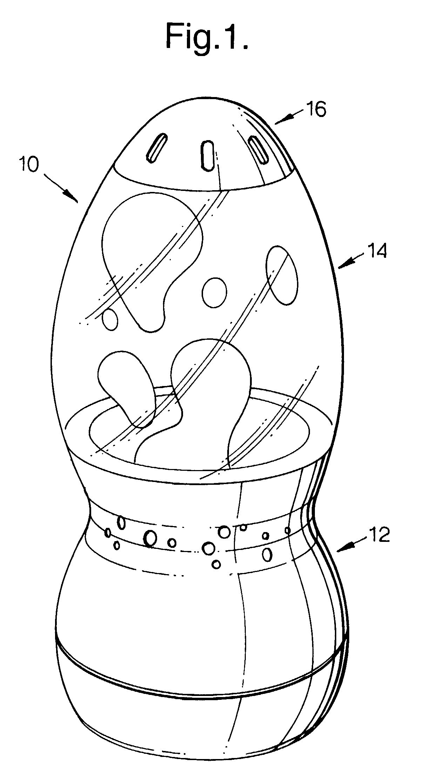

Figure 1 is a perspective view of a fragrance emitting device in operation;
Figure 2 is an exploded view of the fragrance emitting device of Figure 1;
Figure 3 is a vertical cross-sectional view of the device of Figure 2 in operation; and
Figure 4 shows a replaceable cartridge of the device of Figures 1 to 3 being removed from a base section of the device of Figures 1 to 3.

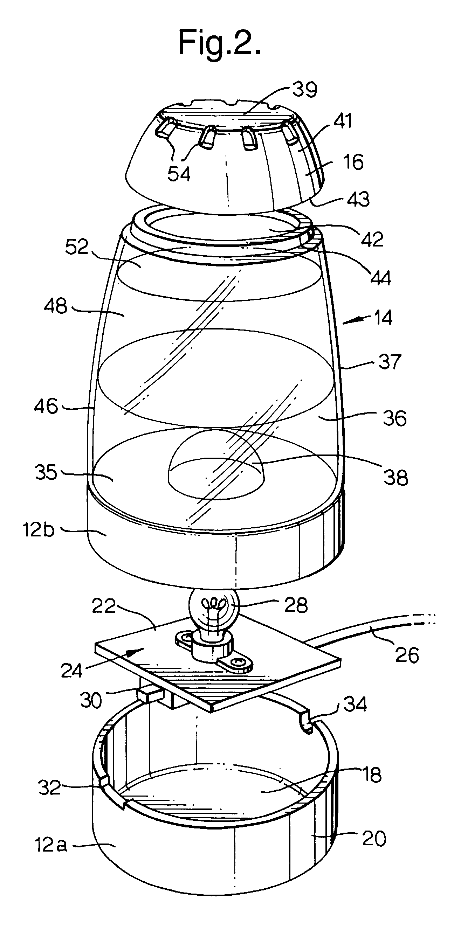

Referring to the Figures, the fragrance emitting device 10 comprises a base 12, a container 14 and a cap 16. Figure 2 shows that base 12 comprises a cup shaped lower part 12a and a generally cylindrical upper part 12b. The lower part 12a has a base 18 and a vertically extending side wall 20. The upper edge of the vertically extending side wall 20 cooperates with and is connected to a corresponding lowermost edge of the upper part 12b. The upper edge of the side wall 20 includes a generally rectangular cut-out 32 and a diametrically opposite semi-circular cut-out 34, for purposes to be described below.

A rectangular platform 22 is located in the base 12 and carries electronic circuitry 24 which is connected by a lead 26 to a power source (not shown). The power source may be mains electricity, but it is to be understood that any suitable power source could be used. A lamp 28 is secured to the platform 22, and is controlled by the electronic circuitry 24 and a switch 30. the electronic circuitry 24 is of known type and will not be described in detail. The switch 30 protrudes through the cut-out 32 in the base side wall 20 for actuation by a user. The lead 26 leaves the base 12 through the semi-circular cut-out 34.

The container 14 comprises a transparent hollow moulding 36 having a base 35 with a dome shaped inwardly extending protrusion 38 in the centre. The base 35 is surrounded by a wall 37 which curves inwardly to an opening 42 at its upper end. The opening 42 is surrounded by a stepped rim 44 for the location of the cap 16 (to be discussed below).

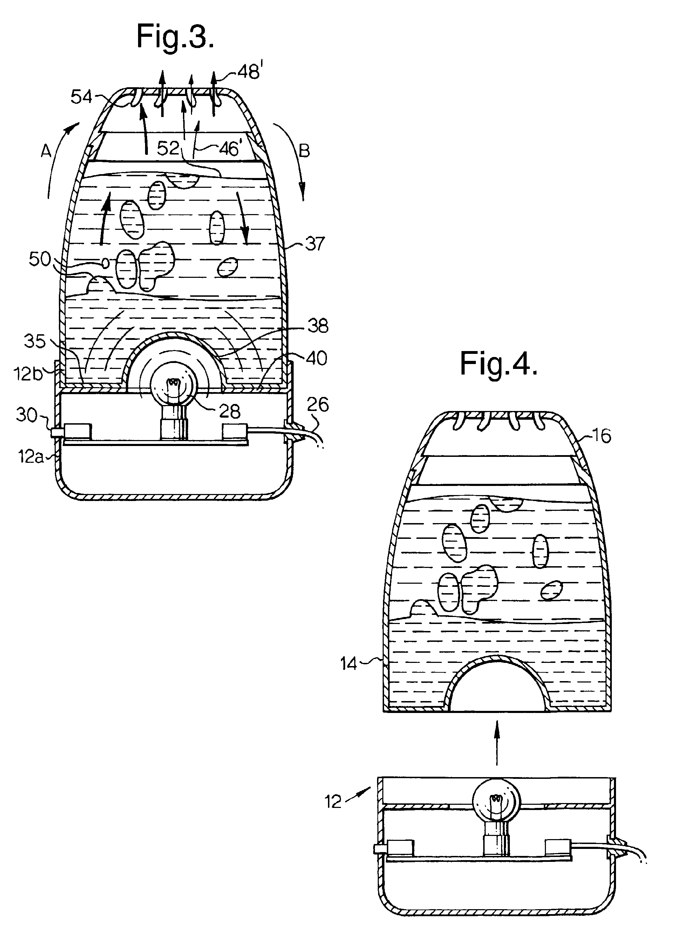

The base 35 of the container 14 sits on a radially inwardly directed annular flange 40 formed on the upper part 12b of the base 12. The lamp 28 protrudes through the open centre of annular flange 40 into the dome shaped protrusion 38 in the moulding 36. This is shown most clearly in Figure 3.

The cap 16 is of inverted cup-shape with an end wall 39 and an outwardly curving surrounding side wall 41. A number of angularly spaced holes 54 extend through the cap 16 at the junction of the end wall 39 and the side wall 41 for a purpose to be described below. A lower edge 43 of the cap side wall 41 sits in the stepped rim 44 of the container 14 to locate the cap 16 on the container 14 with an outer surface of the cap side wall 41 contiguous with the outer surface of the container wall 37.

The container 14 contains a first liquid 46 and a second liquid 48. The liquids 46,48 are immiscible so that first liquid 46 forms a layer at the bottom of container 14 and second liquid 48, of lower density than the first liquid 46, forms a layer directly above the layer formed by the first liquid 46 and has a surface 52 adjacent but below the opening 42 at the upper end of the container 14. The first liquid 46 contains a fragrance.

Referring specifically to Figure 3 which shows the device in operation, the device is operated by use of the switch 30 to connect the lamp 28 to the power source (not shown) via the electronic circuitry 24 and the lead 26. The lamp 28 radiates heat energy through the dome shaped protrusion 38 into the first liquid 46. The dome shape allows a maximum amount of light to pass into the container 14 and maximizes the surface area of the container 14, and thus of the first liquid 46 which is heated by the lamp 28.

When sufficient heat has been transmitted to the first liquid 46, portions 50 of first liquid 46 rise into the layer formed by second liquid 48 in the general direction indicated by arrow A. As the portions 50 rise through second liquid 48, heat is transferred from the portions 50 into the second liquid 48 thereby causing the portions 50 to cool and the second liquid 48 to heat up. This subsequent cooling of the portions 50 causes them to stop rising and eventually to reverse direction and fall back down in a general direction indicated by arrow B into the layer formed by first liquid 46 once more. The returning portions 50 thus recombine with the body of liquid 46 for reheating and a continuation of the process. The portions 50 may comprise discrete bubble-like portions which break away from the layer formed by first liquid 46 or they may comprise more vertically elongate portions which do not break away from the layer formed by first liquid 46.

On first switching on the device 10, the liquids 46,48 will be relatively cool. The initial portions 50 will not reach the surface 52 of the second liquid 48 because the second liquid 48 will be significantly cooler than the first liquid 46, thus the portions 50 of relatively hot liquid 46 will initially be cooled relatively rapidly by second liquid 48. As the process continues, second liquid 48 will heat up due to the heat transfer from the portions 50 and also from the main body of the first liquid 46. As the second liquid 48 increases in temperature the portions 50 will rise increasingly close to the surface 52 until eventually some portions 50 will break the surface 52. This allows the release of fragrance vapours 46' from first liquid 46.

The fragrant vapours 46' rise from the surface 52 through the opening 42, into the space formed by the interior of the cap 16 and out into the atmosphere through the holes 54 in the cap 16. Thus the surrounding atmosphere will be filled with the fragrance. This will continue until the lamp 28 is switched-off using the switch 30. The first liquid 46 will then gradually cease rising through the second liquid 48 until the first liquid 46 reaches a rest position above the base 35 of the container 14. No fragrant vapours are then released.

It will be appreciated that the lamp 28 also transmits light through and out of the wall 37 so providing a light. Of course, both liquids 46,48 may be fragranced and the heating of the second liquid 48 by the first liquid 46 releases this fragrance from the second liquid 48 from the surface 52. Each liquid 46,48 may contain a different fragrance such that a combination of fragrances is emitted in varying rates; a first fragrance being emitted when the portions 50 of first liquid 46 break the evaporation surface and a second fragrance being emitted 48' from more continuous evaporation of second liquid 48 at the surface 52. Equally it could be that only the second liquid 48 is fragranced.

Preferably the liquids 46,48 are differently coloured such that the combination of moving portions 50 of a first colour within a liquid of a second colour and light produces a pleasant visual effect.

It will be understood that it is equally possible to have more than two immiscible liquids in a container 14, one or each of which may be differently coloured and differently fragranced to produce more complex visual and olfactory stimulation.

The device 10 allows easy replacement of a container 14 when all the fragrance or fragrances have been emitted or when it is desired to change the fragrance. Figure 4 shows the container 14 and cap 16 of the device 10 being separated from the base 12. A replacement container 14 and cap 16 are then located on the base 12. This arrangement enables a prolonged life of the device at a minimum cost because there is no need to replace the relatively expensive base 12 containing all of the electronics 24,26,28,30 when the fragrance of the liquids is exhausted. Likewise container 14 could be separated from both the base 12 and the cap 16 to be replaced by another container 14 thus avoiding the unnecessary replacement of cap 16. It would, of course, be just as simple to replace one container 14 and cap 16 with another containing liquids of a different colour and/or fragrance combination if it were desired, even if the previous container 14 and cap 16 had not yet been exhausted.

Another method of replacing the liquids 46,48 would be to remove cap 16 from container 14 in order to pour away the old liquids 46,48 through opening 42 and to refill the container 14 with new liquids 46,48.

It will be appreciated that the fragrance emitting device 10 would work equally effectively as a fragrance emitter if the lamp 28 were replaced by a non-light emitting heat source. In this case there would be no need for the immiscible liquids to be differently coloured, nor would it be necessary for container 12 to comprise a transparent moulding 36.

The opening 42 at the upper end of the container 14 may be covered with a cover such as a membrane that allows vapour to pass through but prevents the liquids spilling.

The cap 16 may contain a fan to dispense the vapours through the holes 54. This may be powered via the electronic circuitry 24.

## Claims

1. A fragrance emitting device (10) **characterised in that** it comprises a heat source (28) and a container (14) containing first (46) and second (48) immiscible liquids, the heat source (28) heating the first immiscible liquid (46) to cause the first liquid to rise from a position at the lower end of the container (14) and pass through the second liquid (48) towards a surface (52) of the second liquid, one of said liquids (46,48) including a fragrance released from said surface (52) by said heated first liquid (46).

2. A device according to claim 1 wherein the first liquid (46) contains a fragrance, the first liquid rising to said surface (52) to release said fragrance.

3. A device according to claim 1 or claim 2 wherein the second liquid (48) contains a fragrance, the heated first liquid (46) causing release of said fragrance from the surface (52).

4. A device according to claim 3 when dependent on claim 2 wherein the fragrance in the first liquid (46) is different from the fragrance in the second liquid (48).

5. A fragrance emitting device according to any one of claims 1 to 4 wherein the container (14) comprises at least partially translucent walls (36) and the device includes a light source (28) so that light from the source passes through said translucent walls (36).

6. A fragrance emitting device according to claim 5, wherein the light source (28) is also the heat source.

7. A fragrance emitting device according to any preceding claim, wherein each of said immiscible liquids (46,48) is differently coloured.

8. A fragrance emitting device according to any preceding claim, wherein the device (10) comprises a base (12) containing the heat source (28) and supporting the container (14), the container being removable from said base.

9. A device according to claim 8, wherein the base (12) is formed in two separable parts (12a, 12b) providing a housing for said heat source (28), one of said parts (12a) containing a supporting surface (20) and the other part (12b) engaging the container (14).

10. A device according to claim 8 or claim 9, wherein the heat source (28) projects from the base (12), the container (14) including a floor (35) having an inwardly directed cavity (38) for receiving the heat source and for providing a heat transfer surface between the heat source (28) and the first liquid (46).

11. A device according to any one of claims 1 to 10, wherein the container (14) includes an open upper end (42) covered by a perforate cap (16) for allowing the passage therethrough of released fragrance (46', 48').
